# EUROPEAN PATENT SPECIFICATION

(11) **EP 4 048 189 B1**
(45) Date of publication and mention of the grant of the patent: **20.09.2023**
(21) Application number: 20753958.6
(22) Date of filing: 14.08.2020
(51) Int. Cl.: A61C 7/08, A61C 19/06, A61F 5/56, A61C 5/00

(54) **BIO-BASED ORTHODONTIC DEVICE AND PROCESS FOR THERMOFORMING THE SAME**
BIOBASIERTE ORTHODONTISCHE VORRICHTUNG UND VERFAHREN ZUM THERMOFORMEN DAVON
DISPOSITIF ORTHODONTIQUE D'ORIGINE BIOLOGIQUE ET SON PROCÉDÉ DE THERMOFORMAGE

(30) Priority: 25.10.2019 EP 19205446
(43) Date of publication of application: 31.08.2022
(73) Proprietor: Bottmedical AG, 4057 Basel (CH)
(72) Inventor: OSMANI, Bekim, 4056 Basel (CH); TÖPPER, Tino, 79112 Freiburg (DE)
(74) Representative: Mertzlufft-Paufler, Cornelius
(86) International application number: PCT/EP2020/072923
(87) International publication number: WO 2021/078422

(56) References cited:
- WO-A1-02/24100
- WO-A1-2015/054101
- US-A1- 2002 051 951
- US-A1- 2017 007 386

## Description

The invention concerns intraoral orthodontic devices to be worn on teeth, as well as a fabrication method for such devices comprising equipping such a device with a releasable substance, which may be a bio-based and/or liquid soluble substance, in particular a bio-based antimicrobial or protective agent.

Intraoral orthodontic devices are known and commonly used in orthodontic therapy for realigning teeth. In this specific application case, the devices are typically designed as a dental splint, which is applied to teeth over several hours. The dental splint exerts forces on the individual teeth of the patient, which results in a realignment of the teeth in directions of desired teeth positions. For this purpose, typically a number of dental splints, which vary slightly from each other in shape, are worn by the patient consecutively to realign the teeth step-wise.

Further known applications of such intraoral orthodontic devices, in particular dental splints, are bleaching of teeth using bleaching pastes which are applied to the teeth by the device, or use of such devices for treating snoring overnight. Intraoral orthodontic devices are also used for avoiding clenching and grinding. Chronic teeth clenching and teeth grinding can cause overuse of the muscles controlling the lower jaw, leading to pain from those muscles. The load on the joint itself can also cause changes inside the joint, leading to pain and limited opening of the mouth.

In all of these applications, providing a high wearing comfort of the device is of central importance for a high acceptance of the treatment by the patient and hence overall success of the therapy.

WO 02 24100 A1 reveals elastic repositioning appliances, which can resiliently reposition teeth from one tooth arrangement to a successive tooth arrangement. The polymeric shell that forms the appliance can hold and dispense therapeutic agents when the appliance is worn in the mouth during orthodontic treatment.

WO 2015 054101 A1 reports on improved metal alloys for forming medical devices such as dental bridges or braces for teeth. This document also discloses the use of a cellulose-based coating that is deposited onto the metal alloy.

In this context, the invention aims at improving the quality of use of intraoral orthodontic devices as introduced in the beginning for the patient. Further, it is an object of the invention to increase the functionality of such devices.

In accordance with the present invention, an intraoral orthodontic devices to be worn on teeth is provided according to claim 1, which solves the afore-mentioned problem. In particular, the invention proposes an intraoral orthodontic device as introduced at the beginning, which may be designed in particular as a dental splint, and which, in addition, is characterized in that the device comprises a core and a cap layer at least partially covering the core; a bio-based material, namely cellulose-based composites or molecules, forms an outer surface of the device and is thus used as the cap layer; and wherein the bio-based material comprises a non-fossil carbon content containing a detectable portion of the carbon isotope ¹⁴C. Furthermore, a melting temperature of the core T_{m,core} is at least 20°C higher than the melting temperature of the cap layer T_{m,cap} such that a compound material is produced at an interface between core and cap layer during thermoforming of the device.

Preferably, the non-fossil carbon content may constitute at least 20%, preferably at least 35%, most preferably more than 50% of the bio-based material. In other words, about 80% to 65% but preferably much less of the bio-based material may be based on other organic material, in particular polymers, derived from fossil sources such as crude oil. The described bio-based material is thus used as a cap layer of the device and it may fully cover a core of the device not made from bio-based material.

¹⁴C is a radioactive isotope produced in the earth's atmosphere at a concentration of about 1.25 x 10⁻¹² = 1250 ppq (ppq = part per quadrillion = 10⁻¹⁵) and incorporated into plants by photosynthesis. If a plant dies, it stops exchanging carbon with its environment, and thereafter the amount of ¹⁴C it contains begins to decrease as the ¹⁴C undergoes radioactive decay. The older a sample containing such biomaterial is, the less ¹⁴C remains to be detected. Hence by detecting the presence of ¹⁴C, material containing non-fossil carbon content can be differentiated from material derived from fossil sources. The presence of the ¹⁴C in a material is thus a direct proof that the material is obtained, at least in part, from non-fossil renewable biological sources. This is because organic polymers derived from fossil oil for example do not show any ¹⁴C any more, since the half-time of radioactive ¹⁴C is less than 6.000 years.

"Detectable portion" may be understood here in that the fraction of the ¹⁴C among all C-atoms of the non-fossil carbon content of the bio-based material may be at least 1 ppq, preferably at least 10 ppq or even at least 100 ppq.

The bio-based material may be further characterized in that it comprises a bio-based content of at least 40%, preferably of at least 50%, and wherein said bio-based content includes the non-fossil carbon content containing ¹⁴C described above as well as nitrogen, oxygen and hydrogen bound to the non-fossil carbon content.

In other words, at least part of the device may be based on one or more bio-based materials. Preferably, however, the outer layers of the device may be fabricated exclusively from bio-based materials.

A bio-based material may be understood here as a material derived from renewable biological resources, i.e., wholly or at least partly derived from natural materials of biological origin such as trees, crops or biomass but not derived from fossil fuels. It can thus be obtained from non-fossil substances derived from living (or once-living) organisms, in particular from plants. Examples of such materials are cellulose-based composites/molecules, in particular cellulose fibers, casein, polyactic acid, or cornstarch or wood-pulp-based materials.

In other words, by fabricating at least part of the device from bio-based materials, the environmental impact of the device in total can be reduced, as the bio-based materials can be easily decomposed. For example, the bio-based material may be a biodegradable material, at least outside of the oral cavity. Inside of the oral cavity, there is no significant amount of UV radiation and in addition, there are no fungi present, which are responsible for the biodegradation observed in normal environments. As a consequence, although the material may be biodegradable, it can be still adapt for prolonged use inside an oral cavity over several weeks, which is a time span typically sufficient for the intended applications of the device described herein. This is particularly true for the material CAB (cf. below).

The outer surface formed by the bio-based material may face the teeth on which the device is worn and/or the oral cavity, when the device is worn on the teeth, i.e., the at least one outer surface can be a top and/or bottom surface of the device. Such a design is of particular advantage for avoiding generation of microplastic particles by abrasion, in particular of polymers derived from fossil oils, and for providing an improved wearing comfort, as will be explained in greater detail below. One advantage of using a bio-based material for the outer surface is that even in case thermoplastic micro particles are generated, they will be based on natural materials and hence will not be harmful to the user of the device.

As an example, one possible (and preferred) embodiment according to the invention is a dental splint fabricated from a sheet of cellulose acetate (CA) or cellulose acetate butyrate (CAB), which may have a thickness in the range of 300 µm to 1000 µm. Such a device can take up liquids and in particular substances such as chemical agents and release them during use in the oral cavity; moreover, such a device offers a very soft and adaptable outer surface, resulting in improved wearing comfort.

Preferably, the cap layer may comprise cellulose acetate butyrate (CAB), which is a bio-based material. For improved wearing comfort and user safety, it is in fact preferable if the cap layer is made up entirely of cellulose acetate butyrate (CAB). Of course, further ingredients such as an agent or a plasticizer may be added to the CAB layer to functionalize it and/or to fine-tune its viscous properties, respectively. For example, the CAB may be applied, in particular together with an agent, as a conformal coating onto the core and the core may not be made from bio-based material but from a polymer derived from fossil sources.

CAB is a water insoluble bio-based material, may be conform with the definition of using C₁₄ content outlined above, and is well suited for dental appliances since it is non-degradable in the oral cavity. In addition, during use, no micro-plastic parts, which may be epigenetically detrimental for human cells, are generated through abrasion of the CAB. This is a great advantage over classic organic polymers which have a tendency to produce such micro-plastic particles. CAB is based on natural cellulose material and thus offers a smooth interface, which improves the wearing comfort. Due to the butyrate content, CAB offers a higher mechanical stability as compared to cellulose acetate (CA) without butyrate content. Important for the user comfort is also that the Young modulus E of CAB is reduced by water uptake below 1 GPa, thus rendering the CAB softer after contact with saliva when the device is worn on the teeth.

According to a preferred embodiment, the molecular mass of the CAB used for the cap layer may be larger than 20.000 g/mol, preferably larger than 30.000 g/mol. Defining the molecular mass is decisive for controlling the mechanical hardness and stability of the CAB layer applied to the core.

The butyryl content of the CAB may be preferably in the range of 10% to 60% by weight. Controlling the butyryl content is decisive for controlling the flexibility of the CAB layer.

Both, the molecular mass of the CAB and its butyryl content are decisive for controlling the speed of release of a releasable agent embedded into the CAB matrix.

A particular high quality of the layer can be obtained if triacetin or polycaprolactone-triol (PCL-T) is mixed into the CAB. These natural softening agents will work as a plasticizer and thus increase the plasticity and/or decrease the viscosity of the CAB. In addition, triacetin or PCL-T will influence the elastic modulus of the cap layer. Other factors influencing the elastic properties of the cap layer are the butyryl content and the molecular weight of the CAB.

Another advantage of using CAB as the cap material of the device is that mixtures of different CAB compositions featuring different lengths of the molecular chains can be used. By optimizing the ratio of each composition, the Young-modulus E as well as the glass transition temperature of the CAB layer may be fine-tuned. Hence, according to the invention the CAB cap layer forming the outer surface of the device may be fabricated by blending CAB compositions of different molecular chain lengths.

In this particular design, the materials used for the core and the cap layer may be different, thus offering more freedom of design, as well as the possibility of tailoring the mechanical and haptic properties of the device, as the core may differ in its mechanical properties from the cap layer. Moreover, the core and the cap layer may be formed from different films or sheets, even if they are formed from the same material.

During use, the teeth come into contact with the outer surface of the device, which is typically worn either on the lower or upper jaw, and material from the cap layer will hence be rubbed of (abrasion). Therefore, forming the cap layer exclusively from bio-based materials has the advantage that during use, only bio-based and thus natural abrasion is produced in the oral cavity. Hence, it can be avoided that a patient's mouth is contaminated with microplastic particles in the form of abrasive particles stemming from non-biological materials such as petroleum-based polymers, which are nowadays frequently used for intraoral orthodontic devices. This is particularly important as there is a risk of uptake of such microplastic particles (in particular sub-micrometer-sized polymeric residues and monomers from the abrased microplastics) into living cells of the patient through the digestive tract.

Using a separate core, which may be a different material than the cap layer, has the advantage that the mechanical properties of the device can be designed independently of the cap layer used, which will be become clear below.

Materials for the cap layer can include a bio-based and biodegradable material such as cellulose acetate (CA), cellulose acetate butyrate (CAB), cellulose acetate propionate (CAP), polylactic acid (PLA), chitosan-based polymers or blends consisting of at least one of them.

Suited as material for the core of the device are bio-based materials such as cellulose acetate, cellulose acetate butyrate, PLA, chitosan or a polyester, a co-polyester, a polycarbonate, a thermoplastic polyurethane, a polypropylene, a polyethylene, a polypropylene and polyethylene copolymer, an acrylic, a cyclic block copolymer, a polyetheretherketone, a polyamide, a polyethylene terephthalate, a polybutylene terephthalate, a polyetherimide, a polyethersulfone, a polytrimethylene terephthalate or a combination thereof (e.g., a blend of at least two of the listed hard polymeric materials). In some embodiments, the core (e.g. hard polymer layer) of the device can include polymeric materials, such as a polycarbonate, a co-polyester, a polyester, and a thermoplastic polyurethane. In some embodiments, the core can be composed of multiple hard layers, e.g., two or three hard polymer layers.

According to the invention, examples of bio-based materials which are exceptionally suited for intraoral orthodontic applications as described herein include: cellulose acetate (CA), in particular cellulose acetate butyrate (CAB); bioplastics, preferably thermoplastic aliphatic polyesters such as polylactide (PLA), in particular based on cornstarch; and natural polysaccharides such as Chitosan.

The invention also proposes an intraoral orthodontic device to be worn on teeth as introduced at the beginning, which may be designed in particular as a dental splint, and which, in addition, is characterized in that the device comprises a core and a cap layer at least partially covering the core, and wherein the cap layer comprises a liquid-absorbing material. This device may have features as described in claim 1 or 2.

The liquid absorbing material can be, for example, a water-absorbing or an oil-absorbing material. In both cases it is preferable if a volumetric liquid absorption ratio, i.e. in particular a volumetric liquid absorption ratio, in particular water absorption ratio, of this material is at least 1.5%, and most preferably at least 2.5%.

The liquid uptake of the liquid absorbing material may be determined with standard test methods, for examples as described in 'ASTM D570 - Standard Test Method for Water Absorption of Plastics'. This method tests the water absorption by drying the specimen under test in an oven for a specified time and temperature and then placing them in a desiccator to cool. Immediately upon cooling, the specimens are weighed. The material is then emerged in water at agreed upon conditions, often 23°C for 24 hours or until equilibrium. After that, specimens are removed, patted dry with a lint free cloth, and weighed again. Besides this preferred method, other methods may be used for determining suited materials, such as impedance measurements as function of relative humidity, nuclear magnetic resonance (NMR) imaging combined with NMR relaxometry, or attenuated total reflection Fourier transform infrared spectroscopy (ATR-FTIR) which can determine for example water diffusion into a polymer film.

By the volumetric liquid absorption ratio values stated above, it is safeguarded that the chosen material is suitable for providing the desired functionality, namely significant liquid absorption to be used as a liquid reservoir and significant deformability, properties which will be discussed in greater detail below.

Providing a component of the cap layer which can absorb water, in particular saliva, is highly beneficial for avoiding tooth decay, in particular by caries, when the device is worn by a patient in the oral cavity. This is because the neutralization or creation of dental plaque is avoided by saliva.

Secondly, using a water-absorbing material at least for the cap layer allows easy integration of substances or agents, in particular temporarily bound substances/agents such as flavor molecules, fluoride molecules or active substances such as drugs, into the material. These substances may be water-soluble such that they can be released into the oral cavity.

In other words, the water-absorbing material may feature water-soluble substances and these substances may be releasable into the oral cavity during wear of the device.

According to another preferred design, the liquid absorbing material may be chosen such that the cap layer's elastic modulus is reduced by at least 10%, but preferably by at least 20%, by absorption of a liquid. This liquid may be in particular water or saliva. Thereby, the device will offer a soft and deformable surface and will hence be comfortable to wear in the oral cavity. For example, based on such a material choice, designs are possible in which the cap layer's elastic modulus is reduced below 0.5 GPa or even below 0.2 GPa after soaking in a liquid, in particular in water, resulting in a very soft touch of the device on the teeth and the gingiva.

Moreover, the ability of the cap layer of absorbing a liquid, in particular water or oil, by swelling is highly useful for improving the adaptiveness of the device to the complex individual topology of a patient's oral cavity. In particular, the softening, i.e. the reduction of the elastic modulus of the cap layer, due to the swelling and liquid uptake provides a mechanism for enabling a comfortable fit of the device to the teeth and gingiva of the patient.

Most preferably, the cap layer may be at least in part, preferably in total, composed of bio-based and water-absorbing materials. Moreover, to avoid skin irritations and allergies it is generally preferred if all materials used for the device are biocompatible.

The intraoral orthodontic device according to the invention can be individually customized to the oral cavity of a patient; it may thus be attached to a human upper or lower jaw in an oral cavity.

For fixing the intraoral orthodontic device to the teeth of a patient, an outer surface of the device may show a topology matched to the topology of the oral cavity of that patient. This outer surface may thus face the teeth to which the device is to be attached to.

The device may thus be used as a dental appliance for positioning a patient's teeth. For this purpose, the device may feature multiple cavities shaped to receive at least some of a patient's teeth. As explained previously, with the device a resilient positioning force may be applied to the patient's teeth.

Devices according to the invention may be used for dental aligning (in particular incremental teeth position and/or orientation adjustment), in particular in the field of aesthetic dentistry, for bleaching teeth, for avoiding teeth grinding at night or teeth clenching or as an anti-snoring device.

In particular for dental alignment applications, a series of the device may be produced with each device of the series slightly approaching the desired final dental alignment. In other words, starting from an unphysiological or anesthetic position of the teeth, the series of devices may be used by a patient to gradually change the position of the teeth into a desired one by using the devices of the series one after another, each time replacing a predecessor device by a successor device after a certain duration of wear, for example after two weeks. The single devices of the series may differ slightly in their shape, thus forcing the teeth stepwise into new positions and/or new orientations.

In particular, the device according to the invention may be designed as a dental splint. A dental splint can be used as an appliance to protect teeth and their supporting structures from damage caused by grinding or clenching. The splint may be in the form of either a tooth night guard or an occlusal splint.

According to the invention, there exist further advantageous embodiments solving the aforementioned problems, which are described in the sub-claims and in the following.

According to a highly preferred embodiment, a core of the device, in particular said core described above, may be made from Polyethylenterephthalat (PET), but most preferably from glycol modified Polyethylenterephthalat (PETG), since the glycol content leads to a higher durability of the PETG (less cracks), which is highly beneficial for excellent formability during thermoforming of the device. This is particularly of advantage when using CAB as the cap layer, since CAB, in particularly when applied in liquid form, for example as a solution based on a solvent such as acetone, to a PETG core can exhibit excellent mechanical interlocking with the PETG.

The underlying reason is that the solvent can soften the core's surface by reducing the interaction of molecular chains of the material of the core. As a result, after treating the core with the solution, the core and the cap layer (or to be more precise, the organic polymer of the cap layer, in particular CAB) can interlock on a nanometer scale, resulting in improved adhesion of the cap layer on the core. After application of the cap layer in liquid form onto the core and interaction of the solvent with the core, the solvent may be simply evaporated thus forming a final solid cap layer. This interlocking is in particular observed when using PETG as the material for the core, acetone as the solvent and CAB as the material for the cap layer.

In addition, as the CAB will soften after prolonged contact with saliva, the PETG core can be important for providing mechanical stability. This also allows reduction of the thickness of the CAB layer(s). Another advantage is that the glass transition temperature T_{g} of CAB is close to that of PETG, which is essential for the thermoforming of the device.

In particular, this may lead to thermal fusion of the cap and core layer during the thermoforming process, which can occur, for example, when the melting temperature of the CAB is reached during thermoforming.

According to a preferred embodiment, the outer cap layer(s) of the device may be functionalized by embedding an agent into the cap layer(s). This agent may be an agent that is releasable from the coating into the oral cavity. The embedding of the agent may be done preferably by adding the agent in the form of an agent liquid to a carrier liquid and forming the cap layer from the mixture of agent liquid and carrier liquid, in particular as a coating applied to a core of the device. This approach safeguards a homogenous distribution of the agent within the cap layer.

According to a highly preferred embodiment, the agent embedded in the cap layer may be derived from a bio-based material as well, most preferably from an essential oil extracted directly from (non-fossil) plants. By this approach, agents such as cinnamaldehyde, lime, thymol, eugenol, linalool, carvacrol, nutmeg, pimenta berry, rosemary, petitgrain, coffee, or anise may be employed as the agent.

Further suited extracts/derivatives obtained from bio-based materials, in particular through extraction from a natural essential oil, which may be used as an agent to be embedded in the coating are α/β-pinene, myristicin, methyl eugenol, menthol, terpinene, p-cymene, linalool, myrcene, pinene, β-ocimene, geranial, sabinene, neral, citronellol, linolenic acid, oleic, stearic, himachalene, bisabolene or trans-cinnamaldehyde.

Another important aspect with regard to the final thermoforming of the device is the thermal stability of the agent. In order to safeguard the proper functionality of the final device, a decomposition temperature of the agent should be at least 50°C above the glass transition temperature T_{g} of the cap layer and/or at least 10°C above the melting temperature Tₘ of the cap layer.

One particular preferred embodiment suggests to employ cinnamaldehyde as an antimicrobial agent and to embed it into a cap layer formed from CAB. This approach is particularly suited as CAB and cinnamaldehyde are highly compatible. Another advantage of using cinnamaldehyde is that it is thermally stable enough for enabling thermoforming of the CAB coating even after embedding the cinnamaldehyde into the CAB.

Particularly suited for avoiding the formation of plaque are cinnamaldehyde and limeone. This is because these agents, which may be embedded into CAB in particular, specifically attack bacteria such as streptococcus mutans and streptococcus mitis.

The antimicrobial activity (AMA) of an agent is generally defined by the hydrophobicity and size of the agent. In case of embedding such an antimicrobial agent into a CAB matrix for example, the more hydrophobic and bigger the agent molecule, the lower will be the release rate of the agent and the lower will be the AMA. On the other hand, using a strongly hydrophobic agent such as carvacrol can result in a slow release rate and thus prolonged time of antimicrobial protection.

All specific agents mentioned above can be released by a diffusion process from a CAB matrix resulting in an asymptotic release over time. The thickness of the CAB coating and the mass percentage of the agent added to the CAB will define the rate of release and hence also the duration of antibacterial protection, in case the agent is an antimicrobial agent such as cinnamaldehyde.

Generally, an agent to be embedded into a CAB matrix should be easy to mix with CAB, i.e., it should not isolate from the CAB as a separate phase in a solution formed from the CAB and the agent, and it should not affect the transparency of the CAB. A side effect of embedding an agent such as cinnamaldehyde can be that it shows some plasticizing effect, such that the content of other plasticizers can be reduced.

In particular, cinnamaldehyde or any other suitable agent may be applied together with a bio-based material, in particular CAB, in liquid form (for example as a homogenous solution of cinnamaldehyde, acetone and CAB) onto the core of the device as a coating. Such a coating will distribute the cinnamaldehyde/the agent evenly over the surface of the device. In this case, it is preferable if the liquid coating is applied and/or solidified prior to thermoforming of the device.

According to a highly preferred embodiment, the cap layer may be plastically deformable, i.e., the cap layer may be in elastically deformable and/or the cap layer may be of a material than can be permanently distorted. This can be the case, for example, if the cap layer comprises entangled fibers. Providing plasticity to the cap layer results in a design, which is very suitable for the applications described herein, as the plasticity results in an improved fit of the device to the teeth and gingiva of the patient and ultimately in improved wearing comfort.

Such plastic deformation may result, in particular, after softening through swelling in a liquid such as water.

As a result, the cap layer may be dimensioned such that an outer surface of the cap layer in contact with the teeth can adapt its shape on a micrometer scale. This may be the outer surface facing the teeth on which the device is worn or the surface facing the teeth which are opposite to the device, for example teeth of the lower jaw when the device is worn on the upper jaw.

The advantage of using a soft and plastically deformable cap layer is an enhanced wearing comfort. This comfort is due to the fact that thanks to this layer, the device surface may adapt to the natural shape of the patient's oral cavity on a micrometer scale, as the cap layer may be deformable on such a size scale. As an important result, the comfort in wearing the device, in particular in case it is designed as a dental splint for realigning teeth, is greatly improved during the first days of wear, as the pressure exerted by the device on the teeth and the gingiva is reduced.

For example, by using materials comprising entangles fibers plastic deformations of at least 20 µm may be achieved using a cap layer of not more than 200 µm, which is concomitant with a relative size change of 10%. The plastic properties or plasticity of the cap layer can be understood here as a deformation of a (solid) material undergoing non-reversible changes of shape in response to applied forces. However, whenever the shape of the oral cavity changes, the cap layer may follow these changes comparable to a piece of metal being re-worked to follow a certain shape.

To further enhance the functionality offered by the device, one particular embodiment suggests that the device features a liquid-soluble, in particular water-soluble or oil-soluble, substance. Such a substance may be a flavor molecule or a drug. The substance can be chosen such that it is releasable through or from the cap layer into an oral cavity of a patient wearing the device. This approach is particularly interesting for providing a pleasant flavor to the device by slowly releasing flavor molecules from the device, when the device is worn by a patient in the oral cavity. Likewise, drugs, for example anti-inflammatory agents - can be released from the device using this approach.

For enabling such added functionality, the invention suggests that the substance is embedded into the device through or into the cap layer prior to use of the device. Thereby, the ease of use for the patient is greatly improved. Such embedding may be done in particular by soaking the device in a liquid solution containing the substance. The liquid embedding, in particular soaking, can be done already during fabrication of sheets from which the device is formed or after forming the device into its final shape.

Another particular design suggests that the device shows a multilayer sandwich structure with the core, which may be of PET, preferably of PETG, being sandwiched between a top and a bottom cap layer, which can be both formed from CAB. In such a design, it is preferable, if the core is fully encapsulated by the top and bottom cap layers. Thus, a large surface can be used for the intended functionality and also microplastic abrasions can be avoided.

The total multilayer sandwich structure may show a maximum thickness of less than 1.5 mm, preferably of even less than 1.0 mm.

A highly preferred design is suggesting the following device structure: a core of PET, preferably of PETG, in particular formed from a medical grade PETG-foil, with a thickness of 300 to 1000 µm, preferably from 400 to 800 um; the core being sandwiched between a top and bottom cap layer formed from CAB, in particular applied in liquid form to the core, and both cap layers with a thickness in the range of 5 to 100 um. Preferably, no intermediate layers are required between the core and the bottom and top cap layers. In addition, it is preferable if the cap layers fully encapsulate the core, in particular such that during use, the patient does not get into contact with the PETG.

In such a design, it is further preferable if the glass transition temperature T_{g,core} of the PETG is in the range 80-100°C and/or if the glass transition temperature T_{g,cap} of the CAB cap layer(s) is/are in the range 80-165°C, most preferably in the range 80-140°C.

The melting range of the CAB material used may be in the range of 120-200°C, depending on the lengths of the molecular CAB chains and the butyryl content.

For intraoral applications, it is of further advantage if the Young Modulus E of the CAB cap layers is lower than the elastic modulus of the core of the device. For example, the Young Modulus E of a PETG core used in the device may be in the range of 0.5 to 2.5 GPa.

According to a preferred design, the top and bottom cap layers may form an outer surface coating, which may have preferably a thickness of less than 0.3 mm.

According to another preferred sandwich structure, the core shows a thickness between 300 µm and 1100 µm, while top and bottom cap layers may show thicknesses between 50 µm and 250 µm, respectively. Here again, the core may be made from PETG and the cap layers from CAB.

When using intermediate layers between the core and the cap layers (see below), it is preferable if these intermediate layers show thicknesses in the range of 10 µm to 100 µm, depending on the application. Intermediate layers may be formed from an elastic material and/or from a material with and elastic modulus that is a factor of 10 lower compared to an elastic modulus of the core.

Moreover, the intermediate layers or layer may be elastic but plastically undeformable.

According to another highly preferred embodiment offering best deformability and shape functionality, the device consists of a five-layer structure with the following layer sequence: bottom cap layer - intermediate layer - core - intermediate layer - top cap layer. Each of these layers may, however, consist of further layers. For example, the core may be formed by laminating or otherwise fusing multiple polymer films.

In another preferred design, that may be used additionally or alternatively, the thicknesses of the top and bottom cap layers are asymmetric. In particular, a top cap layer facing away from the teeth on which the device is worn may be at least 10%, preferably at least 33%, thicker than the bottom cap layer in contact with the teeth on which the device is worn.

More specifically, a top cap layer facing away from the teeth on which the device is worn may show a minimum thickness of 10 µm, whereas a bottom cap layer in contact with the teeth on which the device is worn may show a maximum thickness of 75 µm. Such a design is considered ideal for providing excellent wear comfort due to an adaptiveness of the bottom cap layer, avoiding microplastic contaminations through the use of the top cap layer and allowing a thin overall device design which is comfortable to wear.

In particular, the thicker top cap layer directed towards the oral cavity is optimized for taking up substantial amounts of saliva or other liquids, for example to be released for providing a pleasant taste. In addition, the top cap layer's minimum thickness of 100 µm guarantees protection against abrasion of the inner core layer, which may be of a plastic material. The abrasion of the top cap layer is uncritical, as it is made from a bio-based material. The thinner lower cap layer in direct contact with the teeth on which the device is worn enables a high wearing comfort. At the same time, it's maximum thickness of 75 µm guarantees a proper working of the device, as plastic deformations of the lower cap layer are thus limited. A higher thickness is not necessary in particular for the lower cap layer, as the abrasion of this layer by the teeth is negligible.

When using the sandwich structure as described above, it is generally of advantage for a high wearing comfort and a thin overall design if the bottom cap layer facing the teeth during use of the device is at least as thick as the top cap layer facing away from the teeth.

The core of the device may be a rigid layer providing structural stability and/or shape stability to the device.

In particular, the core may be elastic, preferably with an elastic modulus of at least 0.5 GPa, most preferably with an elastic modulus of at least 1 GPa. The core can thus provide a resilient force for aligning single teeth. Such a design is highly useful, when the device is worn by a patient as a dental splint on her/his teeth.

Preferably for such applications are designs in which an elastic modulus of the core is higher than an elastic modulus of the cap layer, in particular after soaking the device in water. Such a design combines high resilient forces with good adaptiveness of the device.

The core may be formed from a petroleum-based polymer, which are cheap and abundant. Preferably, however, the core may be formed from a bio-based material. As the core has the important function of providing the structural stability to the device, it is generally preferable if the core is thicker than the cap layers and - if used - also the intermediate layers.

As already explained, intermediate layers may be used in between the core and one or more cap layers. In particular, the device may feature an intermediate layer, being softer than the cap layer and/or softer than the core, and separating the core from the cap layer. Separating may be understood here as lying in between the core and cap layer but not necessarily being in direct contact with these layers. For example, in some designs intermediate glue layers or adhesion promotion layers may connect an intermediate layer with another layer.

As already mentioned, it is preferable, if the cap layer is softer than the core, at least after swelling in water.

The intermediate layer or layers may be based for example on silicone or acrylic elastomer or both.

It is to be noted here that the bio-based materials proposed previously for the cap layer may be actually harder than the core, at least in air ambient. Some of these materials, in particular if they can take up significant ratios of water, will soften within the oral cavity as soon as they come into prolonged contact with saliva, a property which is highly beneficial for enhanced wearing comfort of the device. As a result, the previously hard outer cap layer may be rendered softer than the core, after a certain swelling time within the oral cavity. This swelling time can be in the order of a few minutes up to a few hours, depending on the layer thickness.

The intermediate layer is highly beneficial for mitigating stress arising between the core and the cap layer, in particular due to differing thermal expansions. Furthermore, the intermediate layer can balance shear forces arising during thermoforming or deepdrawing of the device. Such shear forces may also arise during water absorption by the cap layer, in which case such an intermediate soft layer will likewise be beneficial. In all of these cases, the intermediate layer may prohibit the generation of air pockets entrapped between the core and the cap layer.

In addition, the intermediate layer may be so soft that it allows elastic deformations thus contributing to an adaptability of the surface of the cap layer to the natural shape of an oral cavity of a patient on a micrometer level and thus further enhancing the wearing comfort.

In particular when using the device for dental aligning, i.e. for re-positioning a patient's teeth, it is of great advantage if a thickness of the cap layer and a thickness of the intermediate layer are limited such that a maximum combined deformation of these two layers after swelling in liquid and due to wearing the device on the teeth is less than 50% of a total thickness of these two layers, preferably less than 50 µm. The underlying reasoning here is that the correct function of the device, in particular a desired realignment of teeth, would be compromised if said maximum combined deformation becomes too large, because in this case, the device could no longer exert relevant positioning forces on the teeth. The plastic deformations of the cap and intermediate layers are a direct result of the softening due to swelling in liquid, in particular in saliva, and the pressure uptake induced by the teeth in contact with the device. Total thickness may be understood here as the sum of the two thicknesses of the cap layer and the soft intermediate layer. The maximum combined deformation may result from elastic and/or plastic deformations of the individual layers. With the proposed design, the device will be comfortable to wear and still useful for repositioning teeth by amounts exceeding 100 µm, for example by 200 to 500 µm, which are typical values used during a step-wise re-positioning of teeth.

At the same time, it may be of advantage if the core shows a volumetric water absorption of less than 0.5%, because this avoids large unintended shape deformations of the device during use.

For example, classic polyethylene terephthalate (PET) is a suitable material for the core, as it shows a very low water absorption ratio.

For the same reason, it may be beneficial if the core features a water-repellant layer and/or is encapsulated by a water-repellant intermediate layer or layers. Such measures will likewise avoid large unintended shape deformations during use by avoiding swelling of the core.

Considering possible fabrication of the device by thermoforming, in particular by deepdrawing, which will be discussed later, it will be of advantage, if a glass transition temperature T_{g,core} of the core and a glass transition temperature T_{g,cap} of the cap layer differ by less than 80°C, preferably by less than 60°C. Such a material choice will result in enough mobility of the molecular chains of both core and cap such that efficient thermal interlocking can be achieved through thermal fusion (sometimes referred to as "thermal welding") between core and cap during the final thermoforming.

In fact, the cap layer(s) may show a glass transition temperature T_{g,cap} which is actually higher than a glass transition temperature T_{g,core} of the core. This may be particularly true if PETG is used as the material for the core and cellulose acetate butyrate (CAB) is used as the material for the cap layer(s).

In addition, a compound material may be produced at the interface between core and cap layer during thermoforming of the device, because the cap layer may exceed its melting temperature, while the core layer is still in the glass transition phase/temperature range. Generally speaking, either the cap layer should reach its melting temperature T_{m,cap} before the core reaches its melting temperature T_{m,core} or the core should reach its melting temperature before the cap reaches its melting temperature. This may be achieved - in particular when considering thermoforming - by safeguarding that the melting temperature of the core T_{m,core} and the melting temperature of the cap T_{m,cap} differ by at least 20°C, preferable by at least 40°C, most preferably by at least 60°C.

Preferably, however, for easy fabrication will be a design in which the melting temperature of the core T_{m,core} is at least 20°C, preferable at least 40°C, most preferably at least 60°C, higher than the melting temperature of the cap T_{m,cap}. In such a design, the cap will become molten while the core material is still solid. For example, according to a preferred embodiment, T_{g,core} may be in the range 80-100°C and T_{g,cap} may be in the range 80-165°C, most preferably in the range 80-140°C. The corresponding melting range of the material used for the cap layer(s) may be in the range of 120-200°C, most preferably in the range 120-180°C. The melting temperature of the core T_{m,core} may be above 200°C. By such a material choice, it is possible to make sure that when heating the device from outside during fabrication, the core will reach T_{g,core} before or when the cap layer reaches T_{g,cap}.

One particular example of such a design is a core made from PETG featuring a glass transition temperature of 80°C and at the same time a melting temperature as high as 210°C. Such a core can be combined with a cap layer based on cellulose acetate butyrate (CAB), which may feature a glass transition temperature in the range of 120°C or above, but at the same time a relatively low melting temperature of 160°C. In other words, in this design the glass transition temperature of the CAB-cap is actually higher than that of the PETG-core, while the melting temperature of the CAB-cap is lower than that of the PETG-core.

Above the glass transition temperature T_{g}, polymers behave like rubbery materials allowing thermoforming, whereas below T_{g} the single molecules of the respective polymer have relatively little mobility, resulting in a glassy or crystalline state with increased rigidity.

Due to the proposed distribution of the glass transition temperatures between core and cap layer, it can be safeguarded when heating the device with an external heat source that as soon as the cap layer reaches its T_{g}, the core layer has already reached its own T_{g}. It can thus be avoided that the core layer is still rigid and not ready for thermoforming when the cap layer is already above its T_{g}.

At the same time, it is highly beneficial if the glass transition temperatures of core and cap layer T_{g,core} and T_{g,cap} differ by less than 80°C, preferably by less than 60°C. This ensures that during thermoforming, both layers are sufficiently mobile to create a sort of thermal fusion which leads to a chemical and/or physical interlock of both layers.

Additionally, the cap layer(s) should reach their/its melting temperature T_{m,cap} before the core reaches its melting temperature T_{m,core}. In other words the melting temperature of the core T_{m,core} should be at least than 20°C, preferable at least 40°C, most preferably at least 60°C higher than the melting temperature of the cap layer (s) T_{m,cap}. As a result, during thermoforming, the cap layer(s) will reach its/their melting temperature T_{m,cap} while the core layer is still in the glass transition phase/temperature range (T_{core} ≈ T_{g,core}).

In fact, a compound material will be produced at the interface between core and the cap layer during thermoforming. This compound material will comprise chains from the organic polymer used for the cap layer and parts of the material used for the core.

Most easily, the device may be fabricated from a 3d-model of an oral cavity, over which the device, in particular said core, is then molded or deepdrawn and/or thermoformed. Following this approach, the 3d-model may be obtained either by a molding process directly from an oral cavity of a patient or it may be based on acquired 3d-data of an oral cavity. Such data can be accurately obtained with a state-of-the-art intraoral optical 3D-scanner.

The core, the cap layer(s), as well as the intermediate layer(s) may be obtained, respectively, from thin films or foils or so-called thermoforming discs. The sandwich structure described previously may thus consist of multiple thin films or layers and be formed into the final shape using heat, pressure and/or vacuum.

According to a preferred embodiment, the device is fabricated by heating a multi-layer-sandwich structure comprising a core and top and bottom outer cap layers, which may have features as described before, from two opposing sides during thermoforming. Following this approach, the core, sandwiched between the top and bottom cap layers, can reach its glass transition temperature before the top and bottom cap layers reach their own glass transition temperatures, respectively. This is of advantage for safeguarding a proper final thermoforming of the device.

Similarly, the device may be fabricated by heating a single film from two opposing sides during thermoforming. This approach is likewise of advantage, as it results in a more uniform heat distribution within the film. In particular when the film is thick, heating from two opposing sides avoids the creation of large temperature gradients within the film, in particular between a top and bottom side of the film, as they are observed when heating the film only from one side. This approach is particularly suited for films comprising or consisting of a bio-based material, as such films tend to turn brownish or yellowish when heating them intensively from one side. Another benefit of the two-sided heating is a faster processing of the film.

Providing a heated source on both sides of the device thus avoids that one of the cap layers is not reaching its glass transition temperature together with the other cap layer, a situation which could occur when heating such a sandwich structure from one side only.

Finally, the device may be at least partly fabricated by an additive-manufacturing process, in particular by 3d-printing of at least the core. Further additive manufacturing techniques besides 3d-printing that may be used for fabricating the device include electron beam melting, stereolithography, selective laser sintering, laminated object manufacturing or fused deposition modeling, to name a few.

We also provide herein a process for thermoforming an intraoral orthodontic device, which may have features as described before. In particular, we propose a process for thermoforming an intraoral orthodontic device, in particular according to one of the claims directed towards such a device, the process being characterized in that a multi-layer-sandwich structure comprising a core and top and bottom outer cap layers is heated from two opposing sides. Needless to say, this multi-layer-sandwich structure may have features as described before with respect to the orthodontic device according to the invention.

Such a two-sided heating may be performed, in particular, such that during heating of the multi-layer-sandwich structure the bottom and top cap layers are synchronously heated towards their glass transition temperatures.

Similarly, this proposed process may also be applied to single films, as previously discussed. In other words, there is provided a process for thermoforming an intraoral orthodontic device, in particular according to one of the claims directed towards such a device, the process being characterized in that a film is heated from two opposing sides. Needless to say, this device may have features as described before with respect to the orthodontic device according to the invention. The two-sided heating of the film may be performed, in particular, such that during heating of the film outer sides of the film are synchronously heated towards their glass transition temperatures. This approach results in a more uniform temperature distribution within the film, with reduced temperature gradients between the outer sides.

For such two-sided heating of a film or a multi-layer-sandwich structure, it is preferable to use a thermoforming apparatus featuring two separate, and preferably individually adjustable, heat sources.

Finally, in accordance with the present invention, there is also provided a process for fabricating an intraoral device and thereby equipping the intraoral orthodontic device, which has features as described before, with a substance releasable into a patient's oral cavity. The substance may be an antimicrobial agent such as cinnamaldehyde or a flavor, a colorant, or a fluoride. This process likewise solves the problem mentioned at the beginning. According to the invention, the process is characterized in that the substance is embedded into a liquid-absorbing, preferably water-absorbing, bio-based cap layer of the device prior to use of the device. This embedding is done by mixing the substance with the bio-based material of the cap layer using a solvent, for example acetone.

The resulting solution may be applied onto the core of the device as a liquid coating. The latter approach is of particular advantage, because the solvent can modify the surface of the core, in particular by reducing the interaction of molecular chains of the material of the core, such that the core and the cap layer can interlock on a nanometer scale, resulting in improved adhesion of the cap layer on the core.

It is further suggested that the cap layer and the core of the device are thermally fused at the interface between cap layer and core during thermoforming of the device thus forming a compound material at the interface. In other words, after thermoforming the device, the cap layer and the core of the device will be thermally fused (a process sometimes referred to as "thermal welding") at the interface between cap layer and core. As a result, a compound material will be formed at the interface. This thermal fusion further improves the mechanical and/or chemical interaction of the cap layer on the core.

As explained previously, the equipping can occur in different stages of the fabrication of the device, in particular during fabrication of a sheet or film, from which the cap layer is formed.

A convenient way of embedding the substance into the cap layer is to soak the device or the cap layer itself in a liquid solution, preferably in a water solution, containing the substance.

The invention thus proposes to use an intraoral orthodontic device featuring a core and cap layer, in particular as explained above, for introducing and releasing a substance such as flavor molecules or a drug into a patient's mouth over a prolonged time span, for example several hours. This approach improves the comfort in wearing the device by providing a pleasant taste to the device through the release of the substance and also delivers a way of providing a drug to the patient's oral cavity in a pleasant and controlled way, in particular with respect to the release rate of the substance.

It is of particular advantage, when the processes described before according to the invention are applied to an intraoral orthodontic device as described before or as defined by one of the claims directed towards such a device.

Preferred embodiments of the present invention will now be described in more detail, although the present invention is not limited to these embodiments: for those skilled in the art it is obvious that further embodiments of the present invention may be obtained by combining features of one or more of the patent claims with each other and/or with one or more features of an embodiment described or illustrated herein.

With reference to the accompanying drawings, where features with corresponding technical function are referenced with same numerals even when these features differ in shape or design:
- Fig. 1: illustrates a fabrication process for a first intraoral orthodontic device according to the invention based on a single film, and
- Fig. 2: illustrates a fabrication process for a second intraoral orthodontic device according to the invention based on a multilayer sandwich structure, and
- Fig. 3: illustrates a preferred embodiment of an orthodontic device according to the invention, and
- Fig. 4: illustrates the definition of non-fossil carbon content and bio-based content used herein to describe bio-based materials.

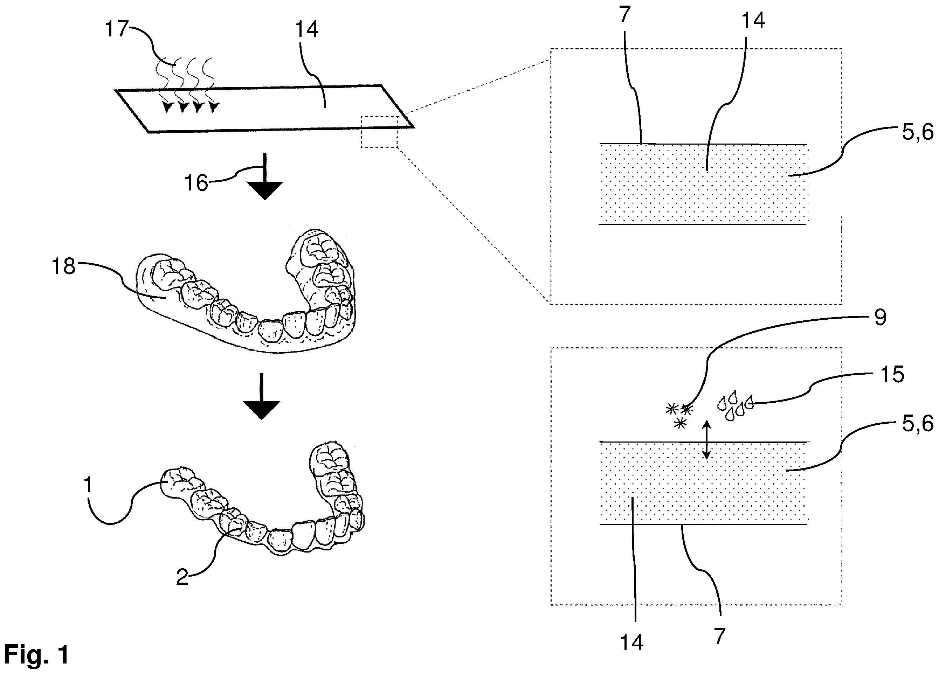

Figure 1 illustrates the fabrication of an intraoral orthodontic device 1 according to the invention, which is shown in the lower left subfigure. As is visible, the device 1 is designed as a dental splint 2 to be worn on teeth inside the oral cavity by a patient. The device 1 is fabricated by first thermoforming 16 a film 14 using heat 17 and vacuum or pressure to achieve a complex 3d-shape fitting the teeth of an upper jaw of an individual patient, as shown in the left center subfigure of Figure 1. For thermoforming of the film 14, a 3d-model 18 is used, as illustrated in figure 1. After thermoforming 16 of the film 14, the resulting 3d-shape is cut into the final shape of the dental splint 2, as visible in the lower left subfigure of Figure 1.

The film 14 consists of a bio-based material 5. This material 5 is rapidly degradable outside of the oral cavity. During wear, however, the device 1 is protected both from UV-radiation and also from fungi, which are responsible for the rapid decay of the bio-based material 5 in normal ambient conditions. Therefore, the device 1 can be worn over several weeks inside the oral cavity by a patient without any significant degradation of performance.

As the device 1 consists entirely of a bio-based material 5, during wear only uncritical natural particles such as fiber fragments are produced, in particular when the teeth of the lower jaw are grinding on the device 1. Thus, the generation of microplastic particles inside the mouth is avoided, which is a problem in state-of-the-art orthodontic devices based on plastic materials.

As indicated by the lower right subfigure in Figure 1, the bio-based material 5 is actually a liquid-absorbing material 6, which can take up a significant volumetric ratio of water, namely more than 2.5%. Hence, it is possible to embed water-soluble substances 9 such as flavor molecules into the material 5, 6. When a patient is wearing the device 1, it will come in contact with saliva, which will then wash-out the flavor molecules over time. As a result, the embedding of the flavor molecules 9 into the liquid-absorbing material 6 prior to use of the device 1 results in a pleasant taste of the device 1 during wear.

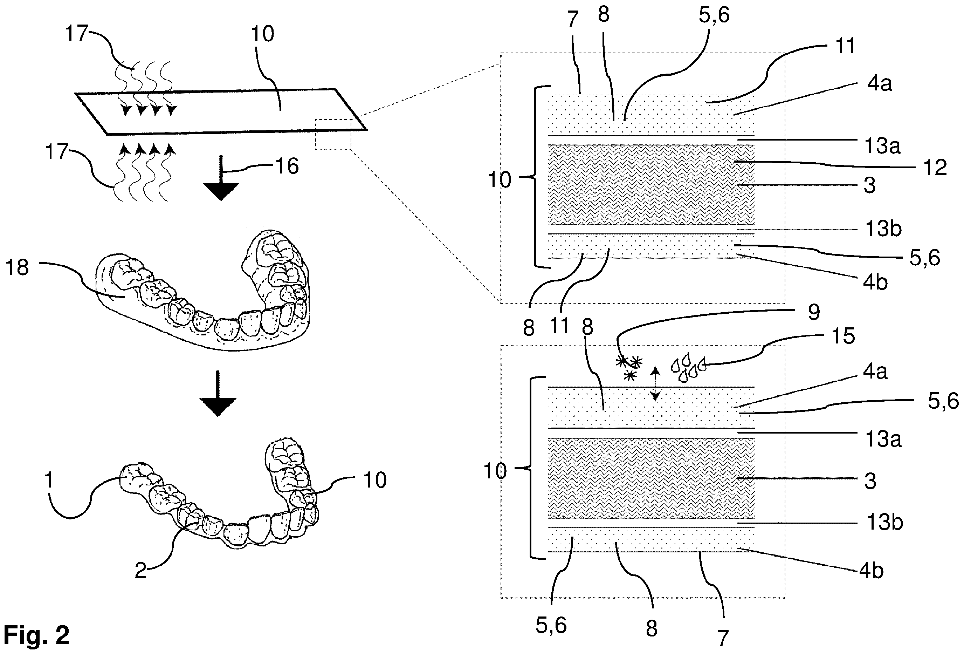

Figure 2 illustrates the fabrication of another example of an intraoral orthodontic device 1 according to the invention, again using a 3d-model 18 being an accurate copy of a topography of a patient's oral cavity. Different from the first example illustrated in figure 1, the device 1 is fabricated not only from a single film 14 but from a complex multilayer sandwich structure 10, whose cross-section is shown on the right half of figure 2. As is visible in the upper cross-sectional view on the right of figure 2, the multilayer sandwich structure 10 consists of a central core 3, which may have a thickness in the range 300-1100 µm, sandwiched between an upper cap layer 4a and a lower cap layer 4b. Each of these cap layers 4a, 4b is separated from the core 3 by an additional soft intermediate layer 13a / 13b.

The core 3 can be made from hard polymers such as polycarbonate or polyurethane. Preferably however, the core 3 is also made from a bio-based and hence biodegradable material. As the core 3 is elastic, with an elastic modulus of 0.5 GPa, it provides structural stability as well as shape stability to the device 1. Hence, when the dentals splint 2 is properly fitted to the teeth of a patient it can exert a resilient force onto single teeth for re-aligning them to a desired position.

The two cap layers 4a, 4b, visible in the cross-sections on the right of Figure 2, consist entirely of a bio-based polymer, comprising cellulose fibers 8 and cellulose acetate (CA). Moreover, the two cap layers 4a, 4b form a coating 11 defining the entire outer surface 7 of the device 1. In other words, the core 3 is fully encapsulated, at least indirectly, by the cap layers 4a, 4b. In fact, the cap layers 4a, 4b can be considered as one uniform outer layer defining the outer surface 7 of the device 1.

As is visible in the cross-sectional views on the right of Figure 2, it is actually preferable for a slim design that is comfortable to wear, if the upper cap layer 4a, which is facing away from the teeth on which the device 1 is worn, is thicker (for example with a thickness in the range of 100 - 300 µm) than the lower cap layer 4b (which may have a thickness in the range 5 - 75 µm), which is in direct contact with the teeth on which the device 1 is worn.

The intermediate layers 13a, 13b are based on a soft polymer such as silicone and hence elastically deformable. Moreover, these layers 13a, 13b are almost impermeable to water (water-repelling). Their thickness can be in the range 10-100 µm.

In contrast, the bio-based material 5 of the cap layers 4a, 4b is a liquid-absorbing material 6, with a volumetric water absorption ratio of more than 2.5%. As a result, the cap layers 4a, 4b are capable of taking up a substantial amount of liquid, and can also change their shape due to swelling in various liquids, in particular in water. Due to the swelling, the outer cap layers 4a, 4b, which are hard polymer layers in air ambient, are rendered soft with their elastic modulus being reduced below 0.5 GPa. As a secondary effect, the outer cap layers 4a, 4b are rendered plastically deformable.

The specific material choice for the cap layers 4a, 4b can also be slightly different, allowing fine-tuning of the thermal and/or mechanical properties of the single layers 4a, 4b. This is particularly relevant for designs, in which the cap layers 4 cover the inner core 3 only partially, which is also possible.

The benefits of this multi-layer-sandwich-design with a stiff inner core 3 and outer bio-based cap layers 4a, 4b are manifold:
Due to the presence of non-crosslinked but entangled fibers 8 in the cap layers 4a, 4b, the outer surface 7 of the device 1 can adapt its shape on a micrometer scale, as the fibers 8 render these layers 4a, 4b plastically deformable, as soon as they have taken up enough liquid.

Due to the stiff core, which may show an elastic modulus being 5 times or even up to a factor of 10 higher than an elastic modulus of the cap layer 4 (after soaking in liquid), the device 1 can be used as a dental splint exerting forces on the teeth. At the same time, the much higher softness and deformability of the outer cap layers 4a, 4b in comparison to the stiff core 3 guarantees a highly comfortable fit. In particular, irritation of the gingiva can be effectively avoided.

As the device 1 is fabricated at least in part from bio-based materials, the environmental impact of the device 1 is reduced, as these materials can be easily decomposed.

As another important advantage, liquid soluble substances 9 such as flavor molecules or drugs, can be embedded into the device, either directly into the liquid-absorbing material 6 or through this material 6 into deeper lying layers of the device. Hence, the device 1 can feature such substances 9 as they can be embedded into the device 1 prior to use.

During use of the device 1 in the mouth, these substances 9 can then be released, either from a cap layer 4, in case the substance is embedded directly into the cap layer 4, or through the cap layer 4, in case the substance 9 is embedded in a deeper-lying layer of the device. A very convenient way of equipping the device 1 with such substances 9 is to soak the device 1 in a liquid containing the desired substance 9. This liquid may be an oil / a water solution containing the substance.

To guarantee, that the intended function of the dental splint 2, namely re-alignment of teeth, is not compromised by an excessive deformability of the cap layers 4a, 4b and the intermediate layers 13a, 13b, the thicknesses of these individual layers 4a, 4b, 13a and 13b can be limited such that a maximum combined deformation, for example a change of the total thickness of the device 1, in reaction to swelling of the outer layers in liquid (in particular when the device 1 is immersed in saliva in the mouth) and due to wearing the device 1 on the teeth is less than 50% of a total thickness of these layers 4a, 4b, 13a and 13b. For example if this combined deformation is below 50 µm, the proper working mechanism of the dental splint can still be guaranteed, as a typical value for a desired re-adjustment of the teeth positions is 200-500 µm per aligner splint.

For the same reason, i.e. to avoid excessive deformations of the total device 1, it is of advantage to limit the amount of water that reaches the inner core 3, in particular if the core 3 is also fabricated from a liquid-absorbing material 6. This may be achieved, for example, by encapsulating the core 3 in water-impermeable layers, which is achieved in the design of figure 2 by using the water-impermeable intermediate layers 13a, 13b.

Concerning the fabrication of the device 1 according to figure 2, it is visible in the upper left subfigure of figure 2 that the 10 multilayer sandwich structure 10 is actually heated from both sides. This approach avoids, that the inner core 3 is still rigid, when one of the cap layers 4a, 4b is already softened because it has reached its own glass transition temperature. For the same reason, the material of the core 3 is chosen such that its glass transition temperature is actually lower than the glass transition temperature of the material 5, 6 used for the cap layers 4a, 4b.

To fully exploit such a design, it is recommended to heat the outer layers of the device 1 synchronously towards their individual glass transition temperatures during thermoforming. This can be safely achieved using two separate and individually adjustable heat 17 sources, placed on the respective side of the device 1.

Therefore, using the two-sided heating approach illustrated in figure 2, the core 3 will reach its glass transition temperature before the cap layers 4a, and 4b reach their own. This means that as soon as the outer cap layers 4a, 4b are rendered soft by the heating, the multilayer sandwich structure 10 will be ready to be shaped by thermoforming. Thermoforming can be done in particular by deep-drawing the sandwich structure 10 over a 3d-model 18 of a patients oral cavity (cf. figure 2). Such a 3d-model 18, which may be cast from plaster or made from a polymer material, can be obtained easily by a casting process or by optically scanning the oral cavity and 3d-printing of the 3d-model 18 based on the obtained data.

Alternatively or additionally, at least part of the device 1, for example the core 3, can be fabricated by additive manufacturing techniques such as 3d-printing. The cap layer 4 and, if necessary an intermediate layer 13, can then be added onto the core 3, using various techniques, including thermoforming and various coating processes.

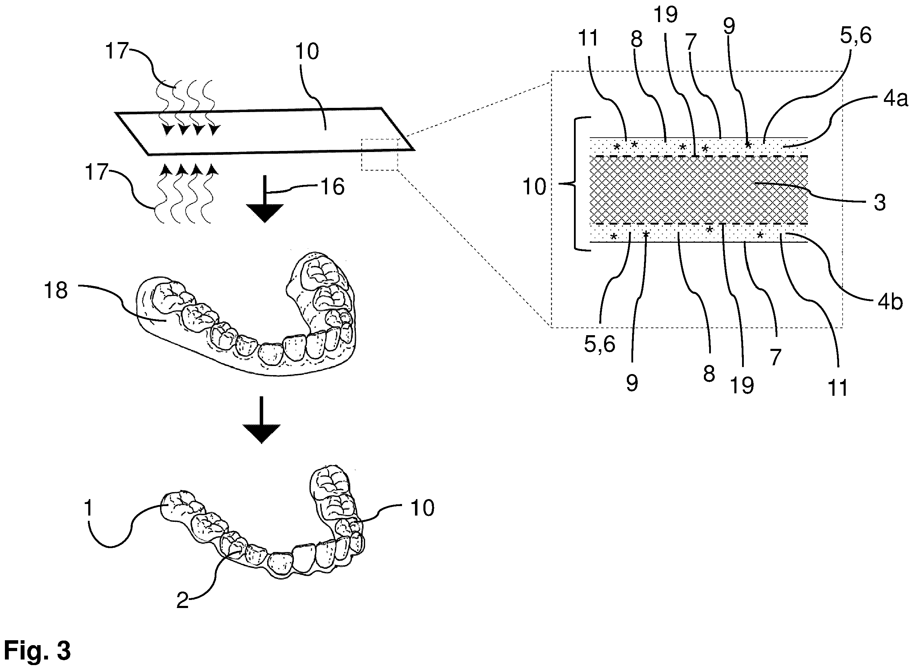

Figure 3 illustrates a preferred embodiment of an orthodontic device according to the invention: the device consists of a multi-layer-structure 10 with a core 3 sandwiched between a top cap layer 4a and a bottom cap layer 4b, forming a coating 11 completely covering the core 3. The layers 4a, 4b are made from cellulose acetate butyrate (CAB). The CAB is first mixed with acetone to yield a carrier liquid.

Next, an agent liquid containing an agent 9, in particular an antimicrobial agent 9 such as cinnamaldehyde and/or an softening agent such as triacetin or polycaprolactone-triol (PCL-T), is mixed with the carrier liquid, which is then applied as a liquid film onto the core 3 made from a glycol modified Polyethylenterephthalat (PETG)-foil with a thickness in the range of 200 to 700 um. After drying of the liquid film (by evaporation of the acetone), a mechanical interlocking occurs at the interface 19, because the acetone softens the PETG surface such that the molecular chains of the CAB can interlock with the PETG on a nanometer scale leading to improved adhesion of the CAB on the PETG.

The resulting CAB coating 11 (on both sides of the PETG-foil) may have a thickness between 5 to 100 um, but preferably between 10 to 50 um, depending on the viscosity of the carrier liquid. The multi-layer-structure 10 is then deepdrawn (by thermoforming) to yield the final orthodontic device 1.

Importantly, during the thermoforming process, a thermal fusion is produced between the CAB and the PETG, which further increases the chemical and/or mechanical interaction of the CAB coating 11 on the core 3. In fact, a compound material will be formed at the interface 19 during thermoforming.

The CAB used for the coating 11 encapsulating the core 3 as illustrated on the right of Figure 3 is in fact a bio-based material 5, as it is produced by blending organic materials derived from fossil sources with organic material, in particular cellulose, derived from non-fossil natural sources such as plants.

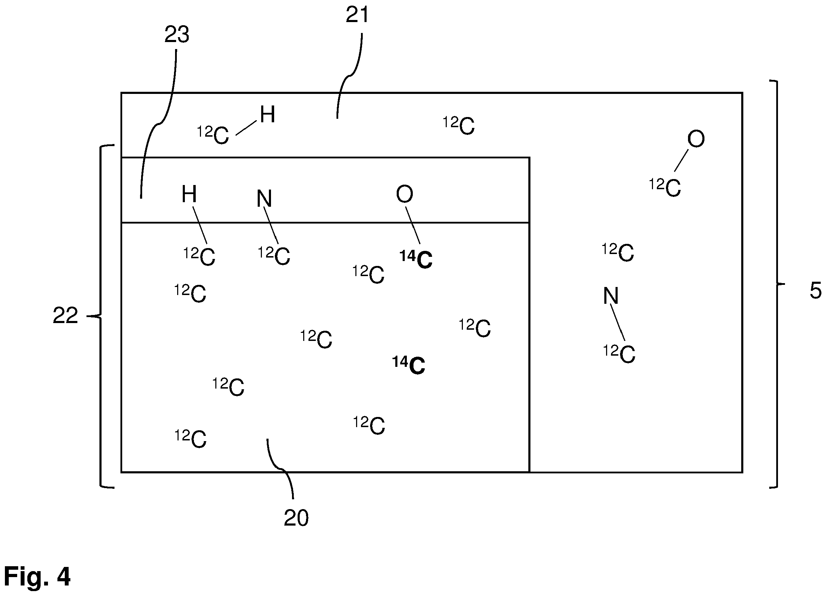

As Figure 4 illustrates schematically, "bio-based material" as understood herein may mean that the material 5 contains a significant portion of non-fossil carbon content 20, for example at least 40%. This non-fossil carbon content 20 is characterized in that it contains the carbon isotope ¹⁴C in a detectable fraction (e.g. more than 10 ppq). There may also be a fossil carbon content 21 (cf. Figure 4) which is based on fossil sources and containing the isotope ¹²C but no detectable fraction of ¹⁴C any more, as the ¹⁴C has been diminished by radioactive decay over thousands of years. As illustrated in Figure 4, there can be defined a further quantity namely the so-called bio-based content 22. This fraction of the material 5 comprises the non-fossil carbon content 20 as well as all hydrogen H-, oxygen O-, and nitrogen N-atoms 23 bound to the non-fossil carbon content 20.

In summary, for improving the quality of use of intraoral orthodontic devices 1 such as dental splints 2 to be worn on teeth as well as for increasing their functionality, it is proposed to fabricate such devices 1 at least in part, but preferably fully from bio-based materials 5. In particular when using a design featuring a cap layer 4 covering an inner stiff core 3 and comprising a bio-based material 5, which can be additionally chosen such that it can take-up a significant amount of liquid by swelling, both the micro-deformability of the device 1 can be improved - resulting in increased wearing comfort for the patient and less microplastic contamination for the patient through fossil-based plastic abrasions - and novel functions can be realized, for example releasing substances 9 such as drugs or flavor molecules or bio-based antimicrobial or protective agents from the device 1.

### List of reference numerals

- 1: intraoral orthodontic device
- 2: dental splint
- 3: core
- 4: cap layer
- 5: bio-based material
- 6: liquid-absorbing material
- 7: outer surface (of 4)
- 8: entangled fibers
- 9: substance/agent (e.g. flavor molecules or drugs)
- 10: multilayer sandwich structure
- 11: coating
- 12: rigid layer
- 13: intermediate layer (e.g. in between 3 and 4)
- 14: film
- 15: liquid
- 16: thermoforming
- 17: heat
- 18: 3d-model
- 19: interface
- 20: non-fossil carbon content (containing ¹⁴C)
- 21: carbon content based on fossil sources (containing no detectable fraction of ¹⁴C any more)
- 22: bio based content (i.e. non-fossil carbon content plus hydrogen, nitrogen, and oxygen bound to this content)
- 23: hydrogen, nitrogen, and oxygen bound to non-fossil carbon

## Claims

1. **Intraoral orthodontic device** (1) to be worn on teeth, in particular designed as a dental splint (2),
**characterized in that**
- the device (1) comprises a core (3) and a cap layer (4) at least partially covering the core (3),
- a bio-based material (5), namely cellulose-based composites or molecules, forms an outer surface (7) of the device (1) and is thus used as the cap layer (4),
- wherein the bio-based material (5) comprises a non-fossil carbon content containing a detectable portion of the carbon isotope ¹⁴C, and
- wherein a melting temperature of the core (3) T_{m,core} is at least 20°C higher than the melting temperature of the cap layer (4) T_{m,cap},
- such that a compound material is produced at an interface between core (3) and cap layer (4) during thermoforming of the device (1).

2. Intraoral orthodontic device (1) to be worn on teeth, according to claim 1,
- wherein the cap layer (4) comprises cellulose acetate butyrate (CAB) (5),
- most preferably wherein the cap layer (4) is made up entirely of cellulose acetate butyrate (CAB) (5).

3. Intraoral orthodontic device (1) according to claim 1 or 2,
- wherein the core (3) of the device (1) is made from Polyethylenterephthalat (PET), preferably from glycol modified Polyethylenterephthalat (PETG).

4. Intraoral orthodontic device (1) according to any one of the preceding claims,
- wherein an agent (9) derived from a bio-based material (5) is embedded into the cap layer (4) and/or
- wherein cinnamaldehyde is embedded into the cap layer (4) / into the bio-based material (5) as an antimicrobial agent (9) and/or
- wherein triacetin or polycaprolactone-triol (PCL-T) is embedded into the cap layer (4) / into the bio-based material (5) as a softening agent (9).

5. Intraoral orthodontic device (1) according to any one of the preceding claims,
- wherein the cap layer (4) comprises a liquid-absorbing material (6) such as CAB,
- in particular with a volumetric liquid absorption ratio of at least 1.5%, preferably of at least 2.5%,
- most preferably wherein the cap layer's (4) elastic modulus is reduced by at least 10%, preferably by at least 20%, by absorption of water.

6. Intraoral orthodontic device (1) according to any one of the preceding claims,
- wherein the device (1) features a liquid-soluble substance (9), such as a flavor or fluoride molecules or a drug, which is releasable through or from the cap layer (4) into an oral cavity of a patient wearing the device (1),
- preferably wherein the substance (9) has been embedded into the device (1) through or into the cap layer (4) prior to use of the device (1).

7. Intraoral orthodontic device (1) according to any one of the preceding claims,
- wherein the device (1) shows a multilayer sandwich structure (10) with the core (3) sandwiched between a top cap layer (4a) and a bottom cap layer (4b),
- preferably wherein the core (3) is fully encapsulated by the top and bottom cap layers (4a, 4b), and/or
- wherein the top and bottom cap layers (4a, 4b) form an outer surface (7) coating (11), preferably of less than 0.3 mm in thickness.

8. Intraoral orthodontic device (1) according to claim 7,
- wherein the thicknesses of the top cap layer (4a) and bottom cap layer (4b) are asymmetric with the top cap layer (4a) facing away from the teeth on which the device (1) is worn,
- in particular wherein the top cap layer (4a) is at least 10% thicker than the bottom cap layer (4b) in contact with the teeth on which the device (1) is worn.

9. Intraoral orthodontic device (1) according to any one of the preceding claims,
- wherein the core (3) is a rigid layer (12) providing structural stability and/or shape stability to the device (1),
- in particular wherein the core (3) is elastic, preferably with an elastic modulus of at least 0.5 GPa, and provides a resilient force for aligning single teeth, when the device (1) is worn by a patient as a dental splint (2) on her/his teeth,
- preferably, wherein an elastic modulus of the core (3) is higher than an elastic modulus of the cap layer (4), in particular after soaking in water.

10. Intraoral orthodontic device (1) according to any one of the preceding claims,
- wherein a glass transition temperature T_{g,core} of the core (3) and a glass transition temperature T_{g,cap} of the cap layer (4) differ by less than 80°C, preferably by less than 60°C.

11. Intraoral orthodontic device (1) according to any one of the preceding claims,
- wherein the device (1) is fabricated by heating a multi-layer-sandwich structure (10) comprising the core (3) and top and bottom outer cap layers (4a, 4b) from two opposing sides during thermoforming.

12. Intraoral orthodontic device (1) according to any one of the preceding claims,
- wherein the device (1) is at least partly fabricated by an additive-manufacturing process, in particular by 3d-printing of at least the core (3).

13. **Process for fabricating an intraoral orthodontic device (1)** according to any one of the claims 1 to 12 and thereby equipping the device (1) with a substance (9), for example an antimicrobial agent (9) such as cinnamaldehyde, which is releasable into a patient's oral cavity, **characterized in that**
- the substance (9) is embedded into a liquid-absorbing bio-based cap layer (4) of the device (1) prior to use of the device (1) by mixing the substance (9) with the bio-based material (5) of the cap layer (4) using a solvent such as acetone, and
- the cap layer (4) and a core (3) of the device (1) are thermally fused at the interface (19) between cap layer (4) and core (3) during thermoforming of the device (1) thus forming a compound material at the interface (19).

14. **Process according to claim 13**
- wherein a multi-layer-sandwich structure (10) comprising the core (3) and top and bottom outer cap layers (4a, 4b) is heated from two opposing sides,
- in particular such that during heating outer sides of the outer cap layers (4a, 4b) of the multi-layer-sandwich structure (10) are synchronously heated towards their glass transition temperatures,
- preferably using a thermoforming apparatus featuring two separate, and preferably individually adjustable, heat sources.

## Patentansprüche

1. **Intraorale orthodontische Vorrichtung (1)** zum Tragen an Zähnen, die insbesondere als Zahnschiene (2) ausgelegt ist, **dadurch gekennzeichnet,**
- **dass** die Vorrichtung (1) einen Kern (3) und eine Kappenschicht (4), die den Kern (3) mindestens teilweise abdeckt, umfasst,
- **dass** ein biobasiertes Material (5), nämlich cellulosebasierte Verbundstoffe oder Moleküle, eine Außenfläche (7) der Vorrichtung (1) bildet und folglich als Kappenschicht (4) verwendet wird,
- wobei das biobasierte Material (5) einen nichtfossilen Kohlenstoffgehalt umfasst, der einen detektierbaren Anteil des Kohlenstoff-Isotops ¹⁴C enthält, und
- **dass** eine Schmelztemperatur des Kerns (3) T_{m,Kern} mindestens 20°C höher als die Schmelztemperatur der Kappenschicht (4) T_{m,Kappe} ist,
- sodass ein Verbundwerkstoff an einer Grenzfläche zwischen dem Kern (3) und der Kappenschicht (4) während des Thermoformens der Vorrichtung (1) erzeugt wird.

2. Intraorale orthodontische Vorrichtung (1) zum Tragen an Zähnen, nach Anspruch 1,
- wobei die Kappenschicht (4) Celluloseacetatbutyrat (CAB) (5) umfasst,
- wobei besonders bevorzugt die Kappenschicht (4) vollständig aus Celluloseacetatbutyrat (CAB) (5) besteht.

3. Intraorale orthodontische Vorrichtung (1) nach Anspruch 1 oder 2,
- wobei der Kern (3) der Vorrichtung (1) aus Polyethylenterephthalat (PET), vorzugsweise aus glykolmodifiziertem Polyethylenterephthalat (PETG) besteht.

4. Intraorale orthodontische Vorrichtung (1) nach einem der vorhergehenden Ansprüche,
- wobei ein Wirkstoff (9), der von einem biobasiertem Material (5) abgeleitet ist, in die Kappenschicht (4) eingebettet ist, und/oder
- wobei Cinnamaldehyd in die Kappenschicht (4) / in das biobasierte Material (5) als ein antimikrobieller Wirkstoff (9) eingebettet ist, und/oder
- wobei Triacetin oder Polycaprolacton-triol (PCL-T) in die Kappenschicht (4) / in das biobasierte Material (5) als ein Weichmacher (9) eingebettet ist.

5. Intraorale orthodontische Vorrichtung (1) nach einem der vorhergehenden Ansprüche,
- wobei die Kappenschicht (4) ein flüssigkeitsabsorbierendes Material (6), wie CAB, umfasst,
- insbesondere mit einem volumetrischen Flüssigkeitsabsorptionsverhältnis von mindestens 1,5%, vorzugsweise mindestens 2,5%,
- wobei besonders bevorzugt der Elastizitätsmodul der Kappenschicht (4) durch Absorption von Wasser um mindestens 10%, vorzugsweise mindestens 20% verringert wird.

6. Intraorale orthodontische Vorrichtung (1) nach einem der vorhergehenden Ansprüche,
- wobei die Vorrichtung (1) eine flüssigkeitslösliche Substanz (9) aufweist, wie etwa ein Aromastoff oder Fluoridmoleküle oder ein Arzneimittel, die durch oder aus der Kappenschicht (4) in eine Mundhöhle eines die Vorrichtung (1) tragenden Patienten freisetzbar ist,
- wobei vorzugsweise die Substanz (9) vor Benutzung der Vorrichtung (1) in die Vorrichtung (1) durch oder in die Kappenschicht (4) eingebettet wurde.

7. Intraorale orthodontische Vorrichtung (1) nach einem der vorhergehenden Ansprüche,
- wobei die Vorrichtung (1) eine mehrschichtige Sandwichstruktur (10) aufweist, wobei der Kern (3) zwischen einer oberen Kappenschicht (4a) und einer unteren Kappenschicht (4b) angeordnet ist,
- wobei vorzugsweise der Kern (3) vollständig von den oberen und unteren Kappenschichten (4a, 4b) eingekapselt wird, und/oder
- wobei die oberen und unteren Kappenschichten (4a, 4b) eine Beschichtung (11) auf der Außenfläche (7) bilden, die vorzugsweise eine Dicke von 0,3 mm aufweist.

8. Intraorale orthodontische Vorrichtung (1) nach Anspruch 7,
- wobei die Dicken der oberen Kappenschicht (4a) und der unteren Kappenschicht (4b) asymmetrisch sind, wobei die obere Kappenschicht (4a) von den Zähnen, auf denen die Vorrichtung (1) getragen wird, abgewandt ist,
- wobei insbesondere die obere Kappenschicht (4a) mindestens 10% dicker als die untere Kappenschicht (4b) in Kontakt mit den Zähnen, auf denen die Vorrichtung (1) getragen wird, ist.

9. Intraorale orthodontische Vorrichtung (1) nach einem der vorhergehenden Ansprüche,
- wobei der Kern (3) eine starre Schicht (12) ist, die der Vorrichtung (1) strukturelle Stabilität und/oder Formstabilität verleiht,
- wobei insbesondere der Kern (3) elastisch ist, vorzugsweise mit einem Elastizitätsmodul von mindestens 0,5 GPa, und eine elastische Kraft zum Ausrichten einzelner Zähne bereitstellt, wenn die Vorrichtung (1) von einem Patienten als Zahnschiene (2) auf seinen Zähnen getragen wird,
- wobei vorzugsweise ein Elastizitätsmodul des Kerns (3) höher als ein Elastizitätsmodul der Kappenschicht (4) ist, insbesondere nach Einweichen in Wasser.

10. Intraorale orthodontische Vorrichtung (1) nach einem der vorhergehenden Ansprüche,
- wobei sich eine Glasübergangstemperatur T_{g,Kern} des Kerns (3) und eine Glasübergangstemperatur T_{g,Kappe} der Kappenschicht (4) um weniger als 80°C, vorzugsweise weniger als 60°C, voneinander unterscheiden.

11. Intraorale orthodontische Vorrichtung (1) nach einem der vorhergehenden Ansprüche,
- wobei die Vorrichtung (1) durch Erhitzen einer mehrschichtigen Sandwichstruktur (10), die den Kern (3) und obere und untere äußere Kappenschichten (4a, 4b) umfasst, von zwei gegenüberliegenden Seiten während des Thermoformens hergestellt wird.

12. Intraorale orthodontische Vorrichtung (1) nach einem der vorhergehenden Ansprüche,
- wobei die Vorrichtung (1) mindestens teilweise mit einem additiven Herstellungsverfahren gefertigt wird, insbesondere mittels 3D-Drucken mindestens des Kerns (3) .

13. **Verfahren zum Herstellen einer intraoralen orthodontischen Vorrichtung (1)** nach einen der Ansprüche 1 bis 12 und dadurch Ausrüsten der Vorrichtung (1) mit einer Substanz (9), beispielsweise mit einem antimikrobiellen Wirkstoff (9) wie Cinnamaldehyd, die/der in die Mundhöhle eines Patienten freisetzbar ist, **dadurch gekennzeichnet, dass**
- die Substanz (9) in eine flüssigkeitsabsorbierende biobasierte Kappenschicht (4) der Vorrichtung (1) vor Benutzung der Vorrichtung (1) eingebettet wird, indem die Substanz (9) mit dem biobasierten Material (5) der Kappenschicht (4) unter Verwendung eines Lösungsmittels wie Aceton gemischt wird, und
- die Kappenschicht (4) und ein Kern (3) der Vorrichtung (1) an der Grenzfläche (19) zwischen der Kappenschicht (4) und dem Kern (3) während des Thermoformens der Vorrichtung (1) thermisch verschmolzen werden, wodurch ein Verbundwerkstoff an der Grenzfläche (19) gebildet wird.

14. Verfahren nach Anspruch 13
- wobei eine mehrschichtige Sandwichstruktur (10), die den Kern (3) und obere und untere äußere Kappenschichten (4a, 4b) umfasst, von zwei gegenüberliegenden Seiten erhitzt wird,
- insbesondere so, dass während des Erhitzens Außenseiten der äußeren Kappenschichten (4a, 4b) der mehrschichtigen Sandwichstruktur (10) synchron auf ihre Glasübergangstemperaturen erhitzt werden,
- vorzugsweise unter Verwendung einer Thermoformungsvorrichtung, die zwei getrennte und vorzugsweise einzeln einstellbare Wärmequellen aufweist.

## Revendications

1. Dispositif orthodontique intra-buccal (1) à porter sur les dents, en particulier conçu sous la forme d'une attelle dentaire (2),
**caractérisé en ce que**
- le dispositif (1) comprend un noyau (3) et une couche de couverture (4) recouvrant au moins partiellement le noyau (3),
- un matériau d'origine biologique (5), à savoir des composites ou des molécules à base de cellulose, forme une surface extérieure (7) du dispositif (1) et est ainsi utilisé en tant que couche de couverture (4),
- dans lequel le matériau d'origine biologique (5) comprend une teneur en carbone non fossile contenant une partie détectable de l'isotope de carbone ¹⁴C, et
- dans lequel une température de fusion du noyau (3) T_{m,core} est supérieure d'au moins 20 °C à la température de fusion de la couche de couverture (4) T_{m,cap},
- de sorte qu'un matériau composé est produit au niveau d'une interface entre le noyau (3) et la couche de couverture (4) pendant le thermoformage du dispositif (1).

2. Dispositif orthodontique intra-buccal (1) à porter sur les dents selon la revendication 1,
- dans lequel la couche de couverture (4) comprend de l'acétobutyrate de cellulose (CAB) (5),
- de préférence, la couche de couverture (4) est entièrement constituée d'acétobutyrate de cellulose (CAB) (5).

3. Dispositif orthodontique intra-buccal (1) selon la revendication 1 ou 2,
- dans lequel le noyau (3) du dispositif (1) est fabriqué à partir de polyéthylentéréphtalate (PET), de préférence à partir de polyéthylentéréphtalate modifié par glycol (PETG).

4. Dispositif orthodontique intra-buccal (1) selon l'une quelconque des revendications précédentes,
- dans lequel un agent (9) dérivé d'un matériau d'origine biologique (5) est incorporé dans la couche de couverture (4) et/ou
- dans lequel du cinnamaldéhyde est incorporé dans la couche de couverture (4)/dans le matériau d'origine biologique (5) en tant qu'agent antimicrobien (9) et/ou
- dans lequel de la triacétine ou du polycaprolactone-triol (PCL-T) est incorporé dans la couche de couverture (4)/dans le matériau d'origine biologique (5) en tant qu'agent adoucissant (9).

5. Dispositif orthodontique intra-buccal (1) selon l'une quelconque des revendications précédentes,
- dans lequel la couche de couverture (4) comprend un matériau absorbant les liquides (6) tel que le CAB,
- en particulier avec un taux d'absorption de liquide volumétrique d'au moins 1,5 %, de préférence d'au moins 2,5 %,
- de la manière la plus préférée, le module d'élasticité de la couche de couverture (4) est réduit d'au moins 10 %, de préférence d'au moins 20 %, par absorption d'eau.

6. Dispositif orthodontique intra-buccal (1) selon l'une quelconque des revendications précédentes,
- dans lequel le dispositif (1) comporte une substance soluble dans un liquide (9), telle qu'un arôme ou des molécules de fluorure ou un médicament, qui peut être libérée à travers ou à partir de la couche de couverture (4) dans une cavité buccale d'un patient portant le dispositif (1),
- de préférence dans lequel la substance (9) a été intégrée dans le dispositif (1) à travers ou dans la couche de couverture (4) avant l'utilisation du dispositif (1).

7. Dispositif orthodontique intra-buccal (1) selon l'une quelconque des revendications précédentes,
- dans lequel le dispositif (1) présente une structure en sandwich multicouche (10) avec le noyau (3) pris en sandwich entre une couche de couverture supérieure (4a) et une couche de couverture inférieure (4b),
- de préférence dans lequel le noyau (3) est entièrement encapsulé par les couches de couverture supérieure et inférieure (4a, 4b), et/ou
- dans lequel les couches de couverture supérieure et inférieure (4a, 4b) forment un revêtement de surface extérieure (7) (11), de préférence d'une épaisseur inférieure à 0,3 mm.

8. Dispositif orthodontique intra-buccal (1) selon la revendication 7,
- dans lequel les épaisseurs de la couche de couverture supérieure (4a) et de la couche de couverture inférieure (4b) sont asymétriques, la couche de couverture supérieure (4a) étant orientée à l'opposé des dents sur lesquelles le dispositif (1) est porté,
- en particulier dans lequel la couche de couverture supérieure (4a) est au moins 10 % plus épaisse que la couche de couverture inférieure (4b) en contact avec les dents sur lesquelles le dispositif (1) est porté.

9. Dispositif orthodontique intra-buccal (1) selon l'une quelconque des revendications précédentes,
- dans lequel le noyau (3) est une couche rigide (12) fournissant une stabilité structurelle et/ou une stabilité de forme au dispositif (1),
- en particulier dans lequel le noyau (3) est élastique, de préférence avec un module d'élasticité d'au moins 0,5 GPa, et fournit une force élastique pour aligner des dents uniques, lorsque le dispositif (1) est porté par un patient comme une attelle dentaire (2) sur ses dents,
- de préférence, un module d'élasticité du noyau (3) est supérieur à un module d'élasticité de la couche de couverture (4), en particulier après trempage dans de l'eau.

10. Dispositif orthodontique intra-buccal (1) selon l'une quelconque des revendications précédentes,
- dans lequel une température de transition vitreuse T_{g,core} du noyau (3) et une température de transition vitreuse T_{g,cap} de la couche de couverture (4) diffèrent de moins de 80°C, de préférence de moins de 60°C.

11. Dispositif orthodontique intra-buccal (1) selon l'une quelconque des revendications précédentes,
- dans lequel le dispositif (1) est fabriqué en chauffant une structure en sandwich multicouche (10) comprenant le noyau (3) et des couches de couverture externes supérieure et inférieure (4a, 4b) à partir de deux côtés opposés pendant le thermoformage.

12. Dispositif orthodontique intra-buccal (1) selon l'une quelconque des revendications précédentes,
- dans lequel le dispositif (1) est au moins partiellement fabriqué par un procédé de fabrication additive, en particulier par impression 3D d'au moins le noyau (3).

13. Procédé pour fabriquer un dispositif orthodontique intra-buccal (1) selon l'une quelconque des revendications 1 à 12 et équiper ainsi le dispositif (1) d'une substance (9), par exemple un agent antimicrobien (9) tel que le cinnamaldéhyde, qui peut être libérée dans la cavité buccale d'un patient, **caractérisé en ce que**
- la substance (9) est incorporée dans une couche de couverture d'origine biologique absorbant les liquides (4) du dispositif (1) avant utilisation du dispositif (1) en mélangeant la substance (9) avec le matériau d'origine biologique (5) de la couche de couverture (4) à l'aide d'un solvant tel que l'acétone, et
- la couche de couverture (4) et un noyau (3) du dispositif (1) sont fusionnés thermiquement au niveau de l'interface (19) entre la couche de couverture (4) et le noyau (3) pendant le thermoformage du dispositif (1), en formant ainsi un matériau composé au niveau de l'interface (19).

14. Procédé selon la revendication 13,
- dans lequel une structure en sandwich multicouche (10) comprenant le noyau (3) et des couches de couverture externes supérieure et inférieure (4a, 4b) est chauffée à partir de deux côtés opposés,
- en particulier de telle sorte que pendant le chauffage, les côtés extérieurs des couches de couverture externes (4a, 4b) de la structure en sandwich multicouche (10) soient chauffés de manière synchrone vers leurs températures de transition vitreuse,
- utiliser de préférence un appareil de thermoformage comportant deux sources de chaleur séparées, et de préférence ajustables individuellement.
